# EUROPEAN PATENT APPLICATION

(11) **EP 4 169 518 A1**
(43) Date of publication of application: **26.04.2023**
(21) Application number: 21842150.1
(22) Date of filing: 16.07.2021
(51) Int. Cl.: A61K 35/28, A61K 38/17, A61K 9/00, A61P 19/02

(54) **COMPOSITION FOR PREVENTING OR TREATING OSTEOARTHRITIS, COMPRISING MESENCHYMAL STEM CELL EXPRESSING TUMOR NECROSIS FACTOR-INDUCIBLE GENE 6**

(30) Priority: 17.07.2020 KR 20200089148
(71) Applicant: Lg Chem, Ltd., Seoul 07336 (KR)
(72) Inventor: RYU, Je Young, Daejeon 34122 (KR); NAM, Seung Woo, Daejeon 34122 (KR); KIM, Chang Young, Daejeon 34122 (KR); KIM, Donghoon, Daejeon 34122 (KR); SHIN, Jung Youn, Daejeon 34122 (KR)
(74) Representative: Hoffmann Eitle
(86) International application number: PCT/KR2021/009190
(87) International publication number: WO 2022/015106

(57) **Abstract**

The present application relates to a use for cartilage regeneration and/or use for osteoarthritis treatment of a mesenchymal stem cell expressing TSG-6 protein. The present application provides a composition for cartilage regeneration and a pharmaceutical composition for osteoarthritis treatment, comprising a mesenchymal stem cell expressing TSG-6 protein as an active ingredient. The composition for cartilage regeneration and/or the pharmaceutical composition for osteoarthritis treatment provided by the present application can increase collagen expression of cartilage cells, reduce inflammation, and restore the cartilage structure.

## Description

### [TECHNICAL FIELD]

### Cross-reference to related applications

The present disclosure claims the benefit of the priority based on Korean patent application No. 10-2020-0089148 filed on July 17, 2020, the entire disclosure of which is herein incorporated by reference.

The present disclosure relates to a composition for treatment of arthritis or a method of treatment using the same, and more specifically, a pharmaceutical composition for treatment of osteoarthritis comprising a mesenchymal stem cell (MSC) expressing a tumor necrosis factor-inducible gene 6 (TSG-6) and/or a method for treatment using the same.

### [BACKGROUND OF THE INVENTION]

Osteoarthritis is also known as degenerative arthritis, and is one of the joint disease that occurs when the cartilage that serves to protect the bone at the end of the bone is worn away. It is the most common type of arthritis, especially in the morning, showing symptoms such as joint pain, swelling and stiffness. Cartilage is a fibrous connective tissue located on the surface of the joint that connects two bones of the body to each other, and is composed of chondrocytes and chondroblasts. The core component of cartilage is collagen, especially collagen II (type 2 collagen, Type II collagen).

Currently, treatment for osteoarthritis focuses on keeping the joint flexible while relieving pain, and drugs and exercise, and physical therapy, and the like are generally used, and when the joint is severely damaged, surgery such as artificial joint replacement is limitedly performed.

TSG-6 is a protein composed of two domains of LINK and CUBE, and is known to be induced by inflammatory stimuli such as TNF and mainly produced in cells such as fibroblasts and connective tissue cells.

TSG-6 is known to have anti-inflammatory action, but when injected into the joint cavity, it is known to be dispersed outside the joint cavity within a short time. According to a report in 2016, the half-life of intra-articularly injected TSG-6 was less than 0.5 hours (Kim D-K, et al., 2016, PLoS ONE 11(1)), and therefore, despite the anti-inflammatory action of TSG-6, there is a limitation in that it is difficult to expect a continuous therapeutic effect during injection.

### [BRIEF SUMMARY OF DISCLOSURE]

### [TECHNICAL PROBLEM]

One embodiment of the present disclosure provides a pharmaceutical composition for cartilage regeneration, comprising a mesenchymal stem cell expressing a TSG-6 protein as an active ingredient.

Another embodiment provides a method of regenerating cartilage, comprising administering a pharmaceutically effective amount of a mesenchymal stem cell expressing a TSG-6 protein to a subject in need of cartilage regeneration.

Other embodiment provides a use of a mesenchymal stem cell expressing a TSG-6 protein in cartilage regeneration or a use of a mesenchymal stem cell expressing a TSG-6 protein in preparation of a pharmaceutical composition for cartilage regeneration.

The effect of cartilage regeneration of the mesenchymal stem cell expressing a TSG-6 protein as above may be due to increase in expression and/or secretion of collagen II protein. In one embodiment, the subject to be applied (administered) with the pharmaceutical composition, method and/or use for cartilage regeneration may be a patient with low expression and/or secretion of collagen II protein (for example, lower than a normal person) and/or a patient in need of increase in expression and/or secretion of collagen II protein. In addition, the method of cartilage regeneration may further comprise identifying a subject in need of cartilage regeneration and/or in need of increase of expression and/or secretion of collagen II protein (for example, having lower expression and/or secretion of collagen II protein than a normal person), prior to the administering step.

Other embodiment provides a pharmaceutical composition for prevention and/or treatment of osteoarthritis, comprising a mesenchymal stem cell expressing a TSG-6 protein as an active ingredient.

Other embodiment provides a method of prevention and/or treatment of osteoarthritis comprising administering a pharmaceutically effective amount of a mesenchymal stem cell expressing a TSG-6 protein to a subject in need of prevention and/or treatment of osteoarthritis.

Other embodiment provides a use of a mesenchymal stem cell expressing a TSG-6 protein in prevention and/or treatment of osteoarthritis, or a use of a mesenchymal stem cell expressing a TSG-6 protein in preparation of a pharmaceutical composition for prevention and/or treatment of osteoarthritis.

The effect of prevention and/or treatment of osteoarthritis of the mesenchymal stem cell expressing a TSG-6 protein as above may be due to cartilage regeneration and/or increase in expression and/or secretion of collagen II protein. In one embodiment, the subject to be applied (administered) with the pharmaceutical composition, method and/or use for prevention and/or treatment of osteoarthritis may be a patient with low expression and/or secretion of collagen II protein (for example, lower than a normal person) and/or a patient in need of increase in expression and/or secretion of collagen II protein and/or cartilage regeneration. In addition, the method for prevention and/or treatment of osteoarthritis may further comprise identifying a subject in need of prevention and/or treatment of osteoarthritis and/or in need of cartilage regeneration, and/or in need of increase in expression and/or secretion of collagen II protein (for example, having lower expression and/or secretion of collagen II protein than a normal person), prior to the administering step.

Other embodiment of the present disclosure provides a composition for expressing and/or secreting collagen II protein or for increasing expression and/or secretion of collagen II protein, comprising a mesenchymal stem cell expressing a TSG-6 protein as an active ingredient.

Other embodiment provides a method of expressing and/or secreting collagen II protein or a method of increasing expression and/or secretion of collagen II protein, comprising administering a pharmaceutically effective amount of a mesenchymal stem cell expressing a TSG-6 protein to a subject in need of increase in expression and/or secretion of collagen II protein.

Other embodiment provides a use of a mesenchymal stem cell expressing a TSG-6 protein in expression and/or secretion of collagen II protein, or increase in expression and/or secretion of collagen II protein, or a use of a mesenchymal stem cell expressing a TSG-6 protein in preparation of a composition for increasing expression and/or secretion of collagen II protein.

### [TECHNICAL SOLUTION]

The present disclosure provides a composition for cartilage regeneration and/or a pharmaceutical composition for prevention or treatment of osteoarthritis and/or a composition for increasing expression and/or secretion of collagen II protein, comprising a mesenchymal stem cell expressing a TSG-6 protein as an active ingredient, and/or a method using thereof.

Herein, "TSG-6 protein" means tumor necrosis factor (TNF)-inducible gene 6 (TNF-inducible gene 6) protein.

As used herein, "mesenchymal stem cell" (MSC) may mean a pluripotent or multipotent cell which can differentiate into various cells, for example, adipocytes, chondrocytes or chondroblasts, osteocytes or osteoblasts, myocytes, and the like. The mesenchymal stem cell is not limited in their origin in the range of maintaining the pluripotency or multipotency. For example, the mesenchymal stem cell may be selected from multipotent cells derived from blood, corium, cord blood, umbilical cord, adipose tissue, placenta, adult muscle, corneal stroma, dental pulp of milk tooth and/or non-marrow tissues such as periosteum, or marrow tissues, preferably, non-marrow tissues, but not limited thereto. Herein, the mesenchymal stem cell may be used interchangeably with "MSC".

The mesenchymal stem cell may be a mesenchymal stem cell derived from a mammal, and the mammal may comprise a human, a dog, a cat, a horse, a mouse, a rat, and the like, but not limited thereto. The mesenchymal stem cell may be derived from an osteoarthritis patient or isolated from a donor other than an osteoarthritis patient.

The composition for cartilage regeneration and/or pharmaceutical composition for prevention or treatment of osteoarthritis and/or composition for increasing expression and/or secretion of collagen II protein, and/or method using thereof provided by the present disclosure comprise or use (administer) a mesenchymal stem cell expressing a TSG-6 protein.

The mesenchymal stem cell expressing a TSG-6 protein may be a mesenchymal stem cell comprising a gene encoding TSG-6 protein, a recombinant vector comprising the gene encoding TSG-6 protein or both of them. The mesenchymal stem cell expressing a TSG-6 protein provided in the present disclosure has a characteristic of expressing TSG-6 protein by comprising a foreign gene encoding TSG-6 protein and/or a recombinant vector (expression vector) comprising the same, and may be distinguished from a cell in which the expression is increased at a gene level and/or a protein level of TSG-6 protein by a stimulating factor such as TNF-a.

In one embodiment, the recombinant vector comprising a gene encoding TSG-6 protein may be one in which a constitutive expression promoter and a gene encoding TSG-6 protein are operably linked to constitutively express the TSG-6 protein, but not limited thereto.

The TSG-6 protein may be protein consisting of the amino acid sequence of SEQ ID NO: 1 or protein encoded by a gene consisting of the nucleic acid sequence of SEQ ID NO: 2.

The expression level of the TSG-6 gene (e.g., mRNA) and/or protein in the mesenchymal stem cell expressing a TSG-6 protein (in which an expression vector of the TSG-6 gene is transduced) may be about 2 times or more, about 5 times or more, about 10 times or more, about 20 times or more, about 50 times or more, about 70 times or more, about 100 times or more, about 200 times or more, or about 300 times or more, compared to the same kind of mesenchymal stem cell (e.g., same kind of naive mesenchymal stem cell, TNF-a induced mesenchymal stem cell, etc.) in which an expression vector of TSG-6 protein gene is not transduced, but not limited thereto.

The TSG-6 protein expression level in the mesenchymal stem cell expressing a TSG-6 protein may be about 10 to about 200 ng/100,000 cells/24 hours (hereinafter, unit same), about 10 to about 100, about 10 to about 80, about 10 to about 70, about 10 to about 65, about 30 to about 200, about 30 to about 100, about 30 to about 80, about 30 to about 70, about 30 to about 65, about 50 to about 200, about 50 to about 100, about 50 to about 80, about 50 to about 70, or about 50 to about 65 ng/100,000 cells/24 hours, but not limited thereto. Herein, the term "about" is an expression to comprise all numerical values in the same or similar range to the numerical value that follows, and it may be a meaning to include a range increased or decreased by about 10%, about 8%, about 5%, about 3%, about 2%, about 1%, about 0.5%, or about 0.1% based on the described numerical value, but not limited thereto (otherwise, hereinafter, it may be interpreted by the same meaning).

The TSG-6 expression level in the composition for cartilage regeneration and/or pharmaceutical composition for prevention or treatment of osteoarthritis provided by the present invention may be about 10 to about 50,000 ng/mL/24 hours (hereinafter, unit same), about 10 to about 30,000; about 10 to about 10,000; about 10 to about 5,000, about 50 to about 50,000, about 50 to about 30,000, about 50 to about 10,000, about 50 to about 5,000, about 100 to about 50,000, about 100 to about 30,000, about 100 to about 10,000, about 100 to about 5,000, about 100 to about 5,000, about 500 to about 50,000, about 500 to about 30,000, about 500 to about 10,000, about 500 to about 5,000, about 1,000 to about 50,000, about 1,000 to about 30,000, about 1,000 to about 10,000, or about 1,000 to about 5,000 ng/mL/24 hours, but not limited thereto.

The mesenchymal stem cell expressing a TSG-6 protein provided by the present invention may express TSG-6 protein continuously, and accordingly, a short half-life in the joint cavity of TSG-6 may be overcome, and therefore it is suitable for recovery (regeneration) of cartilage and/or prevention or treatment of osteoarthritis. In addition, the mesenchymal stem cell expressing a TSG-6 protein provided by the present disclosure has an effect of alleviating inflammation and/or symptoms in osteoarthritis, by inhibiting inflammatory indexes such as TNF-a, IL-1b, and the like, and in addition, increases the expression level and/or secretion level of collagen II protein and regenerates cartilage and/or rebuilds (recovers) the damaged cartilage, thereby allowing more fundamental treatment of osteoarthritis, particularly, degenerative osteoarthritis.

The composition and/or method for cartilage regeneration, and/or pharmaceutical composition and/or method for prevention or treatment of osteoarthritis, and/or composition and/or method for increasing expression and/or secretion of collagen II protein provided by the present disclosure (otherwise, hereinafter, commonly referred to as "composition and/or method provided in the present disclosure") may increase the expression level and/or extracellular secretion at a gene (mRNA) level and/or a protein level of collagen II protein in a chondrocyte and/or cartilage tissue. In one embodiment, the expression level and/or secretion level of collagen II protein in the chondrocyte and/or cartilage tissue in which the composition provided by the present disclosure is administered may be increased, compared to the non-administered control group and/or TNF-a non-treated or TNF-a treated (activated) allogenic mesenchymal stem cell treated group (for example, increased by about 1.2 times or more, about 1.3 times or more, about 1.4 times or more, about 1.5 times or more, about 1.6 times or more, about 1.7 times or more, about 1.8 times or more, or about 1.9 times or more, compared to the collagen II protein expression level (for example, mRNA level and/or protein level) and/or secretion level (for example, collagen II protein concentration in a medium) of the non-administered control group and/or TNF-a non-treated or TNF-a treated allogenic cell treated group; the upper limit of the increase degree is not particularly limited, and for example, it may be about 20 times, about 15 times, about 10 times, about 5 times, or about 2 times, but not limited thereto).

The composition and/or method provided in the present disclosure may maintain and/or recover the cartilage thickness. In one embodiment, the thickness of the cartilage in which the pharmaceutical composition provided by the present disclosure is administered may be increased by over about 1 time to about 10 times or less, over about 1 time to about 5 times or less, over about 1 time to about 3 times or less, over about 1 time to about 2 times or less, about 1.3 to about 10 times, about 1.3 to about 5 times, about 1.3 to about 3 times, about 1.3 to about 2 times, about 1.5 to about 10 times, about 1.5 to about 5 times, about 1.5 to about 3 times or about 1.5 to about 2 times, but not limited thereto. In one embodiment, for the thickness of the cartilage of the OA animal model in which the pharmaceutical composition of the present disclosure is administered, compared to the non-administered OA patient, the recovery of the cartilage thickness was observed, and compared to the same amount of TSG-6 non-expressing MSC administered group, about 1.5 times or more higher cartilage thickness was shown.

The composition and/or method provided in the present disclosure may reduce the expression level of inflammatory indexes inside of chondrocytes and/or synoviocytes and/or inside of cartilage tissue and/or synovial tissue. The reduction of the expression level of inflammatory indexes may be a decrease compared to the non-administered control group that is not administered or applied with the composition and/or method provided in the present disclosure. The inflammatory indexes (e.g., inflammatory cytokine) may be for example, one or more, two or more, three or more or all selected from the group consisting of transforming growth factor-beta 1 (TGF-b 1; TGF-β1), tumor necrosis factor-alpha (TNF-a; TNF-α), interferon-gamma (IFN-r; IFN-γ), and interleukin-6 (IL-6), but not limited thereto. In one embodiment, when a mesenchymal stem cell expressing a TSG-6 protein is administered, the expression level of the inflammatory indexes in synoviocytes and chondrocytes was reduced to the level of the normal group.

In one embodiment, the TGF-b 1 expression level in the cartilage region (chondrocytes and/or cartilage tissue, hereinafter, same) in which the composition and/or method provided in the present disclosure is administered or applied may be at a level of about 55% or less, about 50% or less, about 45% or less, about 40% or less, about 35% or less, or about 30% or less, compared to the TGF-b1 expression level in the patient group, but not limited thereto. The expression level of TGF-b 1 may be reduced as the TSG-6 expression level increases.

The TNF-a expression level in the cartilage region in which the composition and/or method provided in the present disclosure is administered or applied may be at a level of 55% or less, 50% or less, 45% or less, 40% or less, or 35% or less, compared to the TNF-a expression level in the patient group, but not limited thereto. The expression level of TNF-a may be reduced as the TSG-6 expression level increases.

In one embodiment, the IFN-r expression level in the cartilage region in which the composition and/or method provided in the present disclosure is administered or applied may be at a level of about 50% or less, about 45% or less, about 40% or less, or about 35% or less, compared to the IFN-r expression level in the patient group, but not limited thereto. The expression level of IFN-r may be reduced as the TSG-6 expression level increases.

In one embodiment, the IL-6 expression level in the cartilage region in which the composition and/or method provided in the present disclosure is administered or applied may be at a level of about 60% or less, about 55% or less, or about 50% or less, compared to the IL-6 expression level in the patient group, but not limited thereto. The expression level of IL-6 may be reduced as the TSG-6 expression level increases.

In one embodiment, the TGF-b1 expression level in the synovial region (synoviocytes and/or synovial tissue, hereinafter, same) in which the composition and/or method provided in the present disclosure is administered or applied may be at a level of about 50% or less, about 45% or less, about 40% or less, about 35% or less, or about 30% or less, compared to the TGF-b1 expression level in the patient group, but not limited thereto.

In one embodiment, the TNF-a expression level in the synovial region in which the composition and/or method provided in the present disclosure is administered or applied may be at a level of about 45% or less, about 40% or less, about 35% or less, about 30% or less, or about 25% or less, compared to the TNF-a expression level in the patient group, but not limited thereto.

In one embodiment, the IFN-r expression level in the synovial region in which the composition and/or method provided in the present disclosure is administered or applied may be at a level of about 50% or less, about 45% or less, about 40% or less, or about 35% or less, compared to the IFN-r expression level in the patient group, but not limited thereto.

In one embodiment, the IL-6 expression level in the synovial region in which the composition and/or method provided in the present disclosure is administered or applied may be at a level of about 45% or less, about 40% or less, or about 35% or less, compared to the IL-6 expression level in the patient group, but not limited thereto.

The composition and/or method provided in the present invention may comprise or administer a mesenchymal stem cell expressing a pharmaceutically effective amount of TSG-6 alone, or further comprise or administer one or more pharmaceutically acceptable carriers, excipients or diluents. The pharmaceutically effective amount means an amount which can show an effect of cartilage regeneration, prevention, improvement and/or treatment of symptoms of osteoarthritis, and/or increasing expression and/or secretion of collagen II protein, and the total TSG-6 expression level of the mesenchymal stem cell may be about 200 ng/24 hours or more, about 500 ng/24 hours or more , about 1,000 ng/24 hours or more, about 10,000 ng/24 hours or more, about 50,000ng/24 hours or more, about 200 to about 100,000ng/24 hours, about 200 to about 50,0000ng/24 hours, about 200 to about 10,000ng/24 hours, about 200 to about 5,000ng/24 hours, about 200 to about 3000ng/24 hours, about 200 to about 2000ng/24 hours, about 500 to about 100,000ng/24 hours, about 500 to about 50,0000ng/24 hours, about 500 to 10,000ng/24 hours, about 500 to about 5,000ng/24 hours, about 500 to about 3,000ng/24 hours, or about 500 to about 2,000ng/24 hours, but not limited thereto.

In one embodiment, the pharmaceutically effective amount of mesenchymal stem cell may be about 100,000 to about 25,000,000 cells/ml(cell/mL, hereinafter, unit same), about 100,000 to about 20,000,000, about 100,000 to about 15,000,000, about 100,000 to about 10,000,000, about 100,000 to about 5,000,000, about 500,000 to about 25,000,000, about 500,000 to about 20,000,000, about 500,000 to about 15,000,000, about 500,000 to about 10,000,000, about 500,000 to about 5,000,000, about 1,000,000 to about 25,000,000, about 1,000,000 to about 20,000,000, about 1,000,000 to about 15,000,000, about 1,000,000 to about 10,000,000, or about 1,000,000 to about 5,000,000 cells/ml, but not limited thereto.

The "pharmaceutically acceptable" includes compositions that are physiologically acceptable and do not normally cause gastrointestinal disorders, allergic reactions such as dizziness or similar reactions thereto when administered to humans without limitation. The example of the carrier, excipient and diluent may be one or more selected from the group consisting of lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methylhydroxybenzoate, propylhydroxybenzoate, talc, magnesium stearate and mineral oil, but not limited thereto. In addition, a filler, an anti-coagulant, a lubricant, a wetting agent, a flavoring and a preservative, and the like may be further comprised.

The pharmaceutical composition for prevention or treatment of osteoarthritis provided by the present disclosure may be formulated using a method known in the art to provide rapid, continuous or delayed release of an active ingredient. The formulation may be a form of powder, granule, tablet, emulsion, syrup, aerosol, soft or hard gelatin capsule, sterile injection solution or sterile powder. In one embodiment, the composition may be formulated to an injectable solution.

In one embodiment, the pharmaceutical composition for prevention or treatment of osteoarthritis provided by the present disclosure may be administered through various routes including oral, dermal, subcutaneous, intravenous or intramuscular routes for a purpose of treating osteoarthritis, and for example, it may be intra-articularly administered.

The application (administration) subject of the composition and/or method of the present invention may be mammals including primates including humans, monkeys, and the like, rodents including mice, rats, and the like, or cells, tissue derived therefrom or cultures thereof. In one specific example, the subject may be mammals including primates including humans, monkeys, and the like, rodents including mice, rats, and the like, or cells, tissue derived therefrom or cultures thereof, in need of cartilage regeneration, and/or prevention and/or treatment of osteoarthritis, and/or increasing collagen II protein expression.

The present disclosure may also provide a method for preparation of a composition for cartilage regeneration and/or a composition for prevention or treatment of osteoarthritis and/or a composition for increasing collagen II protein expression, comprising transducing a TSG-6 gene and/or a vector comprising the TSG-6 gene into a mesenchymal stem cell.

The transduction may be performed by freely selecting a transduction method known in the art in the range of the purpose of transduction of a mesenchymal stem cell.

The TSG-6 gene and/or vector comprising the same are as described above.

### [ADVANTAGEOUS EFFECTS]

The composition for cartilage regeneration and/or pharmaceutical composition and/or method for prevention or treatment of osteoarthritis provided in the present disclosure have excellent cartilage regenerating ability and/or anti-inflammatory effect.

The composition and/or method provided in the present disclosure may overcome the persistence problem caused by the short intra-articular half-life of TSG-6 protein.

In addition, the composition and/or method provided in the present invention have a fundamental and advantageous effect on cartilage regeneration and prevention and/or treatment of osteoarthritis through this due to an excellent increase effect of the collagen II protein expression level.

### [BRIEF DESCRIPTION OF THE DRAWINGS]

FIG. 1 is a graph of the result of performing ELISA to measure the expression level in selected two kinds of TSG-6 MSC and control group (TSG-6 non-transduced group).
FIG. 2a is a schematic diagram of co-culture experiment design to confirm the collagen expression level for chondrocytes in vitro.
FIG. 2b is a graph showing the quantified values (collagen II protein expression level/GAPDH expression level) of the western blot result confirming the expression level of collagen II and the control group (GADPH) in each group.
FIG. 3a is a microscope photograph confirming the degree of recovering the cartilage tissue by each group in an osteoarthritis rabbit model through safranine O staining (scale bar: 160 um, arrows in -B-: mark clones).
FIG. 3b is graphs of measuring the cartilage thickness by each group in the direction of femur and tibia, respectively, in an osteoarthritis rabbit model and the result showing the statistical significance. n.s.: not significant; *: P<0.05; **: P<0.01; ***: P<0.001; ****: P<0.0001 (hereinafter, same).
FIG. 4a is a photograph of the IHC staining result for TGF-b1 according to each group in the cartilage of the osteoarthritis rabbit model.
FIG. 4b is graphs of measuring the immunoreactivity of TGF-b1 by each group in an osteoarthritis rabbit model and the result showing the statistical significance.
FIG. 5a is a photograph of the IHC staining result for TNF-a according to each group in the cartilage of the osteoarthritis rabbit model.
FIG. 5b is graphs of measuring the immunoreactivity of TNF-a by each group in an osteoarthritis rabbit model and the result showing the statistical significance.
FIG. 6a is a photograph of the IHC staining result for IFN-r according to each group in the cartilage of the osteoarthritis rabbit model.
FIG. 6b is graphs of measuring the immunoreactivity of IFN-r by each group in an osteoarthritis rabbit model and the result showing the statistical significance.
FIG. 7a is photographs of the IHC staining result for IL-6 according to each group in the cartilage of the osteoarthritis rabbit model.
FIG. 7b is graphs of measuring the immunoreactivity of IL-6 by each group in an osteoarthritis rabbit model and the result showing the statistical significance.
FIG. 8a is photographs of the result of performing H&E staining for each group in the synovial membrane of the osteoarthritis rabbit model.
FIG. 8b is graphs showing the thickness of the synovial epithelial cells and the number of inflammatory cells as the result of H&E staining for each group in the synovial membrane of the osteoarthritis rabbit model and statistical significance thereof.
FIG. 9a is microscope photographs of the result of performing IHC staining for TGF-b 1 and TNF-a for each group in the synovial membrane of the osteoarthritis rabbit model.
FIG. 9b is graphs of the result of measuring the immunoreactivity for TGF-b 1 and TNF-a for each group in the synovial membrane of the osteoarthritis rabbit model and statistical significance thereof.
FIG. 10a is microscope photographs of the result of performing IHC staining for IFN-r and IL-6 for each group in the synovial membrane of the osteoarthritis rabbit model.
FIG. 10b is graphs of the result of measuring the immunoreactivity for IFN-r and IL-6 for each group in the synovial membrane of the osteoarthritis rabbit model and statistical significance thereof.
FIG. 11a is a graph showing the TSG-6 mRNA expression level of naive MSC and TNF-a treated MSC and TSG-6 expression vector transduced MSC.
FIG. 11b is a graph showing the TSG-6 protein expression level of naive MSC and TNF-a treated MSC and TSG-6 expression vector transduced MSC.
FIG. 11c is a graph showing the Col II mRNA expression level of chondrocyte co-cultured with naive MSC and TNF-a treated MSC, or TSG-6 expression vector transduced MSC.

### [MODE FOR INVENTION]

Hereinafter, the present disclosure will be described in more detail by examples. However, the following examples are intended to illustrate the present disclosure only, but the scope of the present disclosure is not limited by the following examples.

Unless otherwise mentioned herein, "AC" is an abbreviation of articular surface lining cartilage, and "OA" is an abbreviation of osteoarthritis.

### Example 1. Production of TSG-6 gene transduced mesenchymal stem cell

### 1-1. Preparation of lentivirus

After preforming codon optimization for TSG-6 cDNA sequence (Genbank accession no. NM_007115; SEQ ID NO: 3), a lentivirus vector comprising cDNA sequence (SEQ ID NO: 4) completing the codon optimization by requesting to SIRION-Biotech was prepared.

### 1-2. TSG-6 gene transduction to mesenchymal stem cell

As a mesenchymal stem cell (MSC), cord blood-derived mesenchymal stem cells were cultured in a medium for MSC culture under the conditions of 5%(v/v) CO₂, 37 degrees Celsius. To the cultured MSC 50,000 cells or 100,000 cells, a lentivirus in which the TSG-6 cDNA was transduced was added and they were cultured under the conditions of 5%(v/v) CO₂, 37 degrees Celsius for 24 hours again. The MSC in which the cDNA sequence of TSG-6 gene (TSG-6 MSC) was selected by ELISA.

### Example 2. Confirmation of TSG-6 protein secretion level in TSG-6 MSC

For confirmation of the TSG-6 protein secretion level in TSG-6 MSC prepared in Example 1, the TSG-6 protein secretion level was confirmed by performing ELISA. Specifically, as a control group, MSCs uninfected with a lentivirus comprising TSG-6 cDNA were used, and each MSC was cultured in a serum-free medium under the culture conditions of 5% CO₂, 37 °C for 24 hours to measure the total amount of TSG-6 protein expressed for 24 hours.

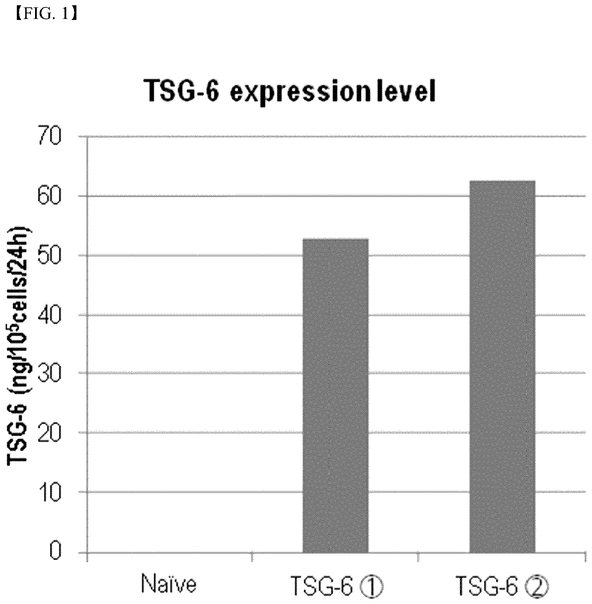

In FIG. 1, a graph of the result of measuring the TSG-6 expression level in the TSG-6 MSC and control group was shown. As the result of measurement, it was confirmed that the TSG-6 transduced mesenchymal stem cell secreted about 10 to 100ng/100,000 cells/24 hours of TSG-6 protein.

### Example 3. Confirmation of effect of treating osteoarthritis of TSG-6 MSC in cartilage-synoviocyte co-culture model

To confirm the therapeutic effect for osteoarthritis of TSG-6 MSC at an *in vitro* level, the expression level of collagen II depending on addition of the TSG-6 MSC prepared in Example 1 under the cartilage-synoviocyte co-culture condition was confirmed.

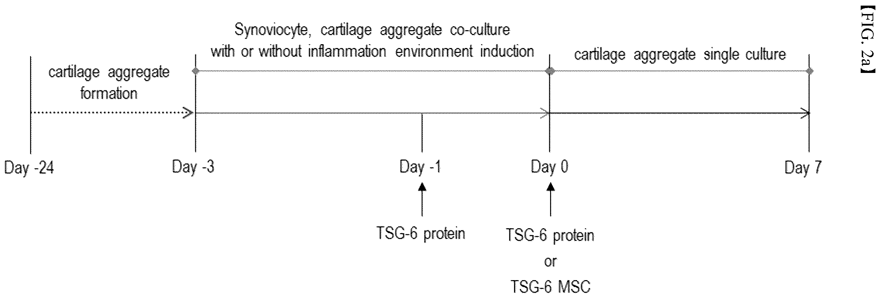

In FIG. 2a, a schematic diagram of the experimental design was shown. Specifically, chondrocytes were inoculated in a U-bottom 96 well plate alone and cultured in a cartilage differentiation medium at 5%(v/v) CO₂, 37 °C for 3 weeks (21 days) to form a cartilage aggregate, and this cartilage aggregate was co-cultured with synoviocytes at 5% CO₂, 37 °C for 2 days, and then inflammation was induced by adding TNF-alpha (TNF-a) 10ng/ml and interleukin 1-beta (IL1b) 10ng/mL.

After 2 days of co-culture (inflammation induction), purified TSG-6 protein 200ng/mL was added and cultured for 1 day, and then the synoviocytes were removed and the purified TSG-6 protein of 200ng/mL was added as same as the concentration added in advance and cultured for 7 days again. For the experimental group to confirm the therapeutic effect for osteoarthritis of TSG-6 MSC, synoviocytes were removed after 3 days of co-culture and to express TSG-6 protein at a level of 10ng/mL/24 hours or to express TSG-6 protein at a level of 30ng/mL/24 hours, they were co-cultured for 7 days again at different amounts of TSG-6 MSC.

As a normal group, an inflammation non-induced group (No inflammation control, NIC) and as a negative control group, a group without addition of TSG-6 after inducing inflammation (Inflammation control, IC) were used. After that, using western blot, the expression level of collagen II protein by each group was measured, and as a control group, the expression level of GAPDH protein was confirmed.

In Table 1 below, each group was arranged and shown.

**[Table 1]**

| Numb er | Name | Infla mmati on | TSG-6 MSC | purified TSG-6 |
|---|---|---|---|---|
| 1 | NIC | X | X | X |
| 2 | IC | O | X | X |
| 3 | TSG6 200 | O | X | TSG-6 concentration 200ng/mL |
| 4 | MSC 10 | O | TSG-6 expression level 10ng/mL/24hr (5×10⁴ TSG-6 transduced MSCs) | X |
| 5 | MSC 30 | O | TSG-6 expression level 30ng/mL/24hr (1.5×10⁵ TSG-6 transduced MSCs) | X |

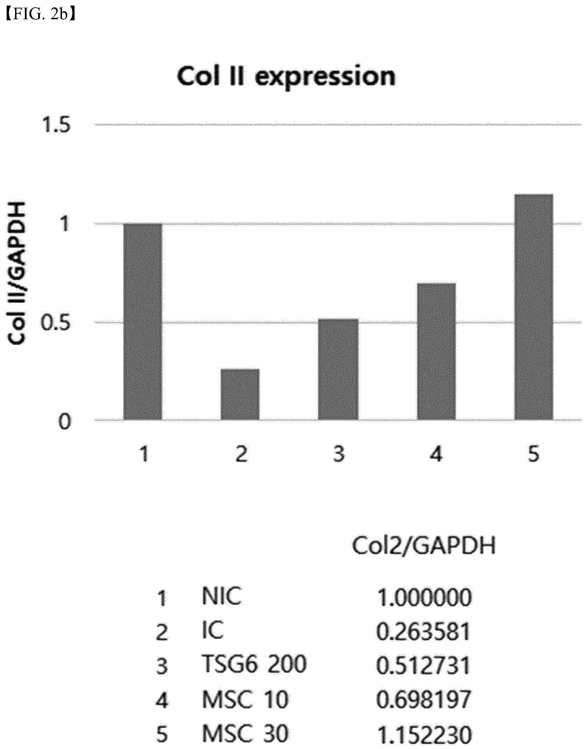

In FIG. 2b, the values (collagen II protein expression level/GAPDH expression level) of quantifying the western blot result by each group were shown.

As shown in FIG. 2b, collagen II protein was hardly produced in the inflammation-induced chondrocytes (column 2), but when added in a form in which TSG-6 protein was expressed in purified protein or TSG-6 MSC, the expression level of collagen II protein was increased in both. In addition, in both cases that the TSG-6 protein was added in a form of purified protein and it was added in a MSC form in which the TSG-6 expression vector was transduced, the expression level of collagen II protein increased depending on the TSG-6 protein concentration, but in particular, when the TSG-6 protein was added in a MSC form in which the TSG-6 expression vector (namely, a form that the TSG-6 protein was expressed in MSC) was transduced, compared to the case that the purified TSG-6 protein was added, the expression level of collagen II protein was higher, and when co-cultured with TSG-6 MSC expressing TSG-6 protein at a level of 30ng/mL/24 hours, the expression level of collagen II protein at a similar level to NIC group was shown.

In other words, it was confirmed that the expression level of collagen II protein increased depending on the TSG-6 protein concentration, and the TSG-6 MSC expressing TSG-6 protein provided by the present disclosure had an effect of recovery of collagen II protein at a similar or more excellent level than purified TSG-6.

Through the corresponding result, it can be confirmed that the TSG-6 MSC provided by the present disclosure has an effect of cartilage regeneration and/or recovery, and thereby, it can be seen that it may be advantageously used for treatment of arthritis.

### Example 4. Confirmation of therapeutic effect of TSG-6 MSC in degenerative osteoarthritis rabbit model

In order to confirm the therapeutic effect of TSG-6 MSC at an *in vivo* level, an experiment was performed in a degenerative osteoarthritis rabbit model.

Specifically, as the degenerative osteoarthritis rabbit model, female SPF New Zealand White rabbits (Kangda) (about 10 months old) were used, and during administration of TSG-6 MSC, and the like, the body weight was about 3.9kg. The rabbit model was divided into 6 groups in total. Brief description for each group was represented in Table 2 below.

**[Table 2]**

| Numb er | Name | O A | Description |
|---|---|---|---|
| A | Intact | X | Normal group |
| B | OA | O | Negative control group (administering only an excipient) |
| C | TSG-6 I | O | TSG-6 MSC 6.9x10⁵ cells administration group (TSG-6 concentration 200ng/24h) |
| D | TSG-6 II | O | TSG-6 MSC 6.9x10⁶ cells administration group (TSG-6 concentration 2000ng/24h) |
| E | Naive I | O | TSG-6 non-expressing MSC 6.9x10⁵ cells administration group |
| F | Naive II | O | TSG-6 non-expressing MSC 6.9x10⁶ cells administration group |

To C to F groups among 6 groups represented in the Table 2, the corresponding MSC was injected using a syringe, respectively. In 16 weeks after injection, a knee j oint was harvested from each rabbit and the cartilage tissue was subjected to hematoxylin-eosin (H&E) staining, IHC staining or safranin O staining, and microscope observation and each immunoreactivity, measurement of epithelial thickness or analysis of the number of inflammatory cells was performed.

### 4-1. Cartilage structure index confirmation (safranin O staining)

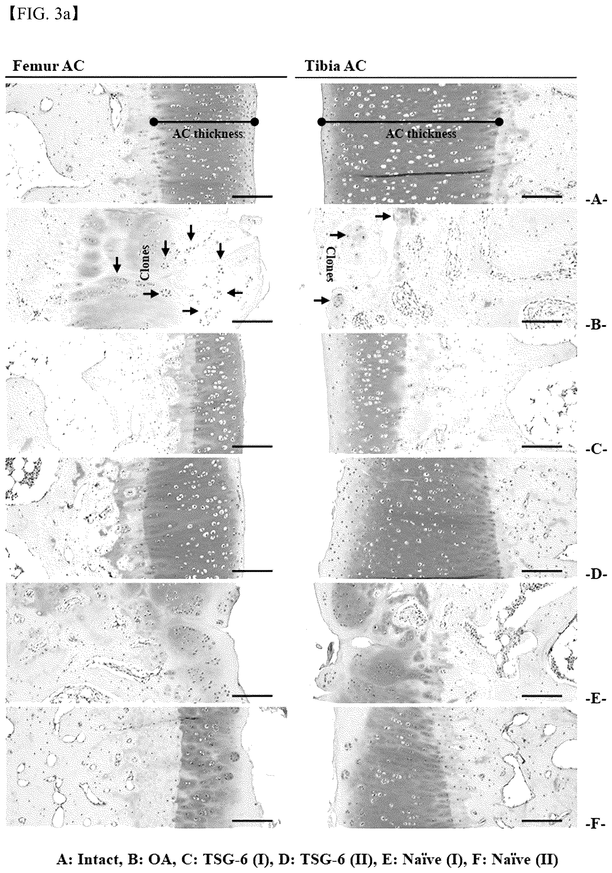

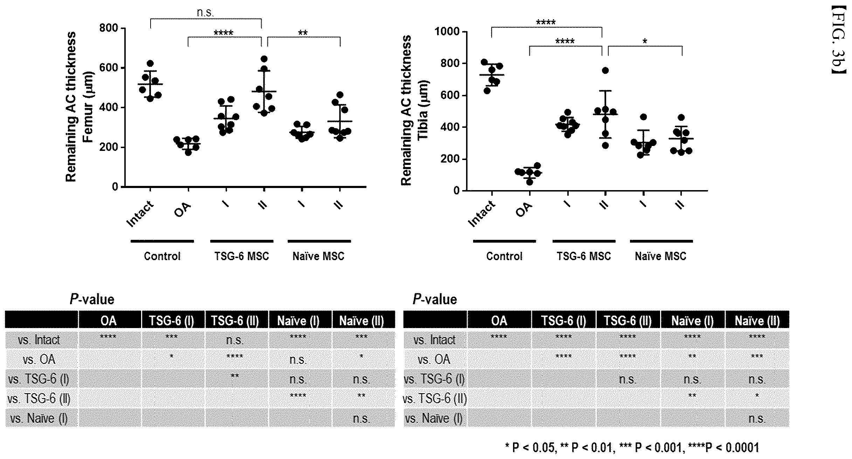

The photograph of the result of safranin O staining was shown in FIG. 3a, and the graph of the result of measuring the thickness of the articular surface lining cartilage (AC) by each group on the side of femur and tibia, respectively, and the statistical significance were shown in FIG. 3b.

The average values of the measured AC thickness were shown, respectively, in Table 3 below. Femur means the AC thickness measured on the side of femur, and Tibia means the AC thickness measured on the side of tibia.

**[Table 3]**

| Group | Femur(um) | Tibia(um) |
|---|---|---|
| Intact | 518.65 | 728.36 |
| OA | 218.19 | 114.10 |
| TSG-6 I | 345.64 | 417.73 |
| TSG-6 II | 481.00 | 480.91 |
| Naive I | 275.51 | 304.28 |
| Naive II | 331.45 | 328.84 |

All in the osteoarthritis (OA) groups, compared to the normal group, the cartilage thickness was significantly reduced, and in TSG-6 I and TSG-6 II groups, the cartilage thickness was significantly recovered, respectively, and in particular, as the concentration of TSG-6 protein increased, the increase in cartilage thickness tended to increase. This concentration dependence was more remarkably shown in the cartilage on the side of femur. In TSG-6 II group, in particular, the improvement of the cartilage structure was confirmed to the extent that there was no significant difference (n.s.: not significant) from the normal group. On the other hand, in Naïve groups, a significant difference was not observed between Naive I and Naive II groups.

### 4-2. Inflammatory index confirmation through cartilage IHC staining

In the cartilage tissue of each group of Table 2, immunohistochemistry staining (IHC staining) was performed for TGF-b1, TNF-a, IFN-r, and IL-6, respectively, to confirm the expression level of each inflammatory index, respectively.

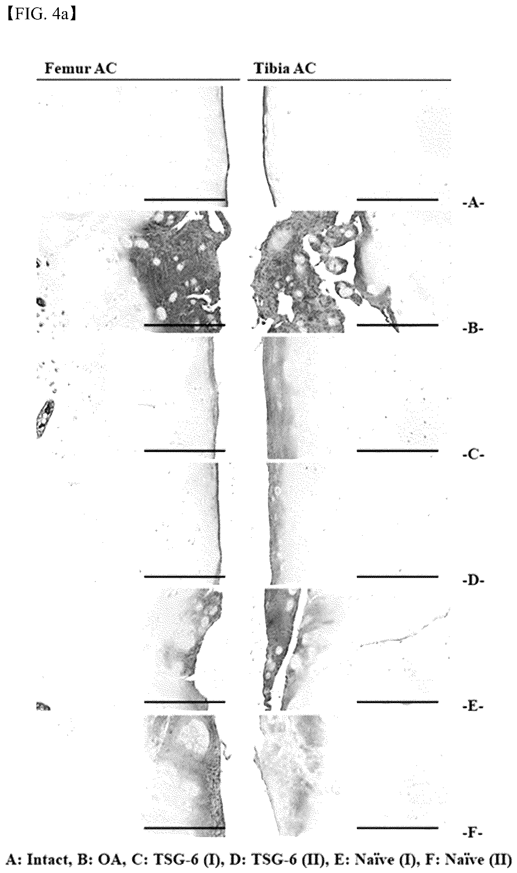

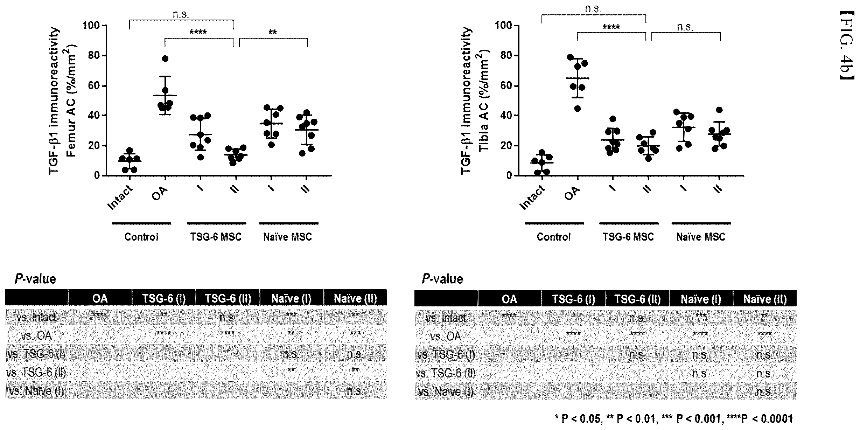

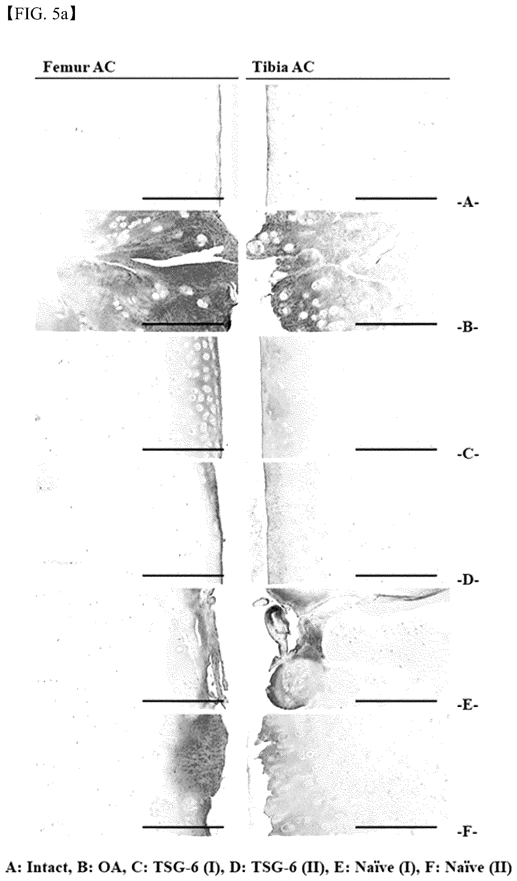

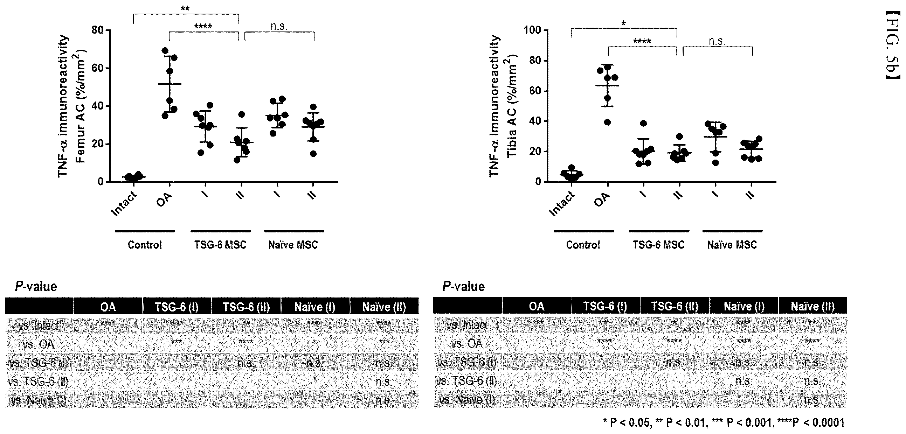

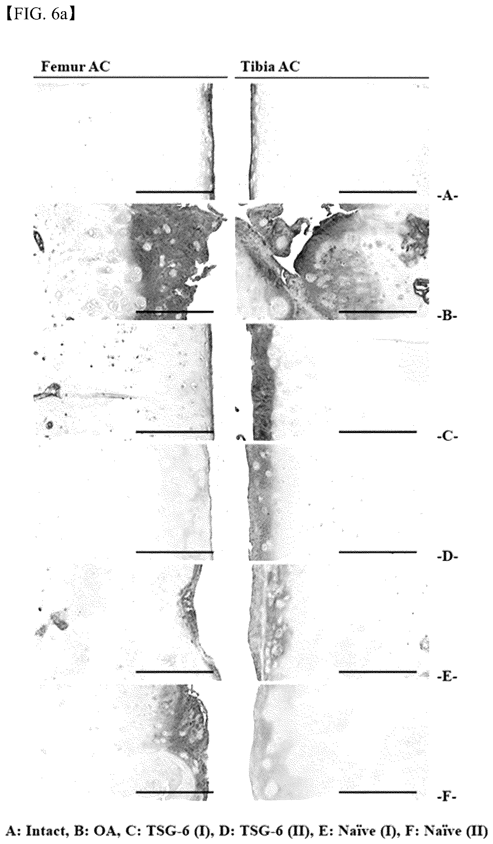

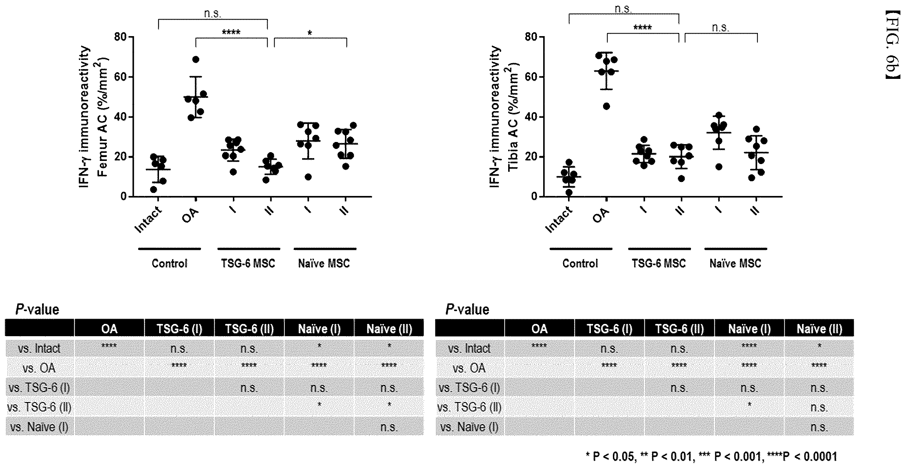

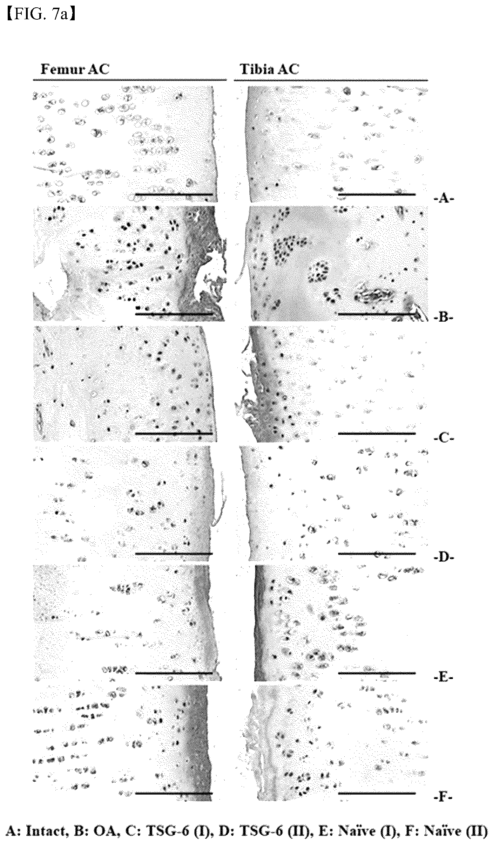

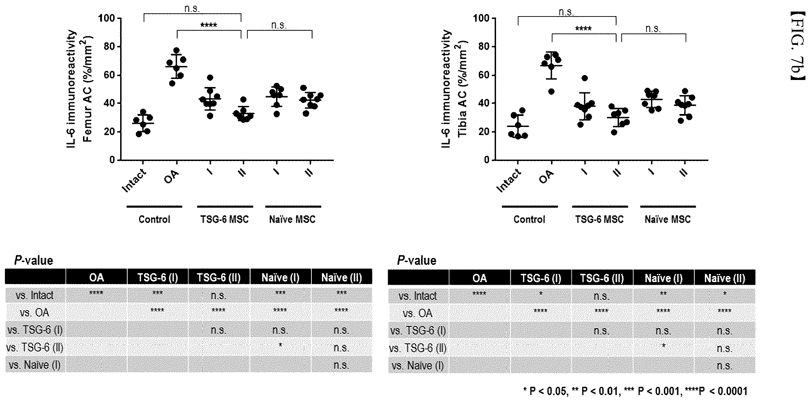

The IHC staining result and immunoreactivity of each index measured on the side of femur and tibia were shown in FIG. 4a to FIG. 4b for TGF-b1, in FIG. 5a to FIG. 5b for TNF-a, in FIG. 6a to FIG. 6b for IFN-r and in FIG. 7a to FIG. 7b for IL-6, and the measurement values of each immunoreactivity were shown in Table 4 below. In Table 4 below, the numerical unit (%/mm²) of each inflammatory index expression level was omitted.

**[Table 4]**

| Inflammatory index | TGF-b 1 | | TNF-a | | IFN-r | | IL-6 | |
|---|---|---|---|---|---|---|---|---|
| Region | Femur | Tibia | Femur | Tibia | Femur | Tibia | Femur | Tibia |
| Intact | 9.82 | 8.61 | 2.72 | 4.82 | 13.73 | 10.03 | 26.22 | 24.10 |
| OA | 53.62 | 65.11 | 51.73 | 63.68 | 50.06 | 63.15 | 66.16 | 66.89 |
| TSG-6 I | 27.63 | 24.06 | 29.29 | 20.26 | 23.44 | 21.49 | 43.28 | 38.11 |
| TSG-6 II | 14.07 | 20.08 | 20.94 | 19.26 | 15.14 | 20.17 | 32.99 | 30.19 |
| Naive I | 34.88 | 32.47 | 35.13 | 29.68 | 28.04 | 32.16 | 44.92 | 42.94 |
| Naive II | 30.68 | 27.84 | 29.12 | 21.69 | 26.60 | 22.18 | 42.34 | 38.76 |

In the OA group, the immunoreactivity of TGF-b1, TNF-a, IFN-r, and IL-6 was shown very high, but in all the TSG-6 MSC treatment groups, compared to the OA group, low immunoreactivity was shown. In particular, for TGF-b1, IFN-r, and IL-6, the immunoreactivity was reduced to the extent similar to the normal group (n.s.), and therefore, it was confirmed that the TSG-6 MSC provided by the present disclosure showed an excellent anti-inflammatory effect.

In addition, in the TSG-6 MSC treatment groups of the present disclosure, as the treatment dose increased, all of the numerical values of the immunoreactivity of the inflammatory index decreased, compared to the Naive groups showing a similar inflammatory index numerical value regardless of the administration dose, and thus it was confirmed that the anti-inflammatory activity was excellent.

### 4-3. Inflammatory index confirmation through synovial H&E staining

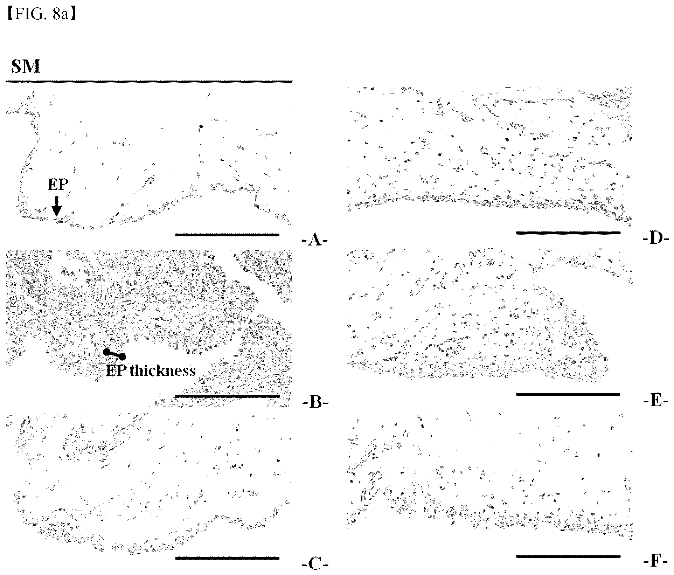

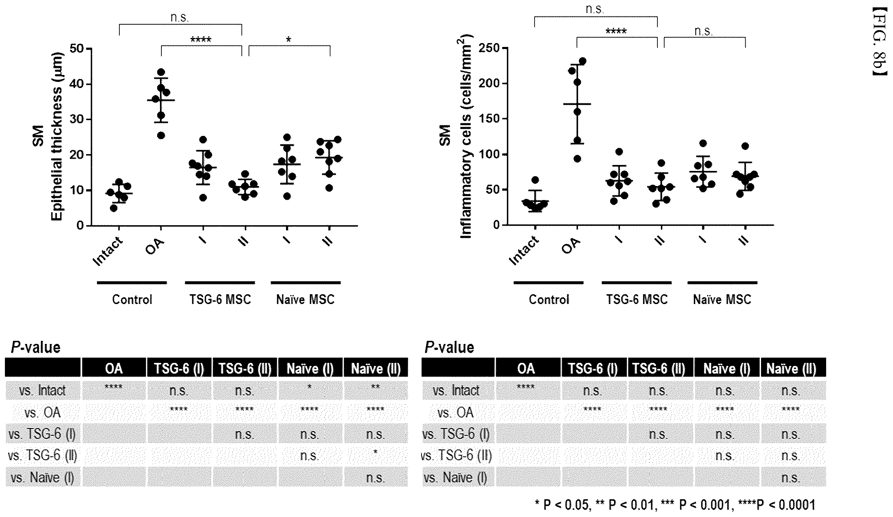

The synovial membrane of each group of Table 2 was isolated and H&E staining was performed, and the result was shown in FIG. 8a to FIG. 8b. FIG. 8a is a graph showing the synovial membrane epithelial thickness and a table showing its statistical significance and FIG. 8b is a graph showing the number of inflammatory cells per unit area and a table showing its statistical significance. In Table 5 below, the epithelial thickness measured in the synovial membrane and the number of inflammatory cells (IF cell) were shown, respectively.

**[Table 5]**

| Group | Synovial membrane epithelial thickness (um) | Number of inflammatory cells (cells/mm²) |
|---|---|---|
| Intact | 9.15 | 34.00 |
| OA | 35.43 | 171.00 |
| TSG-6 I | 16.51 | 62.75 |
| TSG-6 II | 11.00 | 54.29 |
| Naive I | 17.39 | 75.71 |
| Naive II | 19.33 | 69.00 |

As could be confirmed in FIG. 8b, the synovial membrane epithelial thickness was reduced to the extent similar to Intact group from TSG-6 II group, and the number of inflammatory cells was also reduced to the extent similar to Intact group.

### 4-4. Inflammatory index confirmation through synovial IHC staining

In the synovial tissue of each group of Table 2, immunohistochemistry staining (IHC staining) was performed for TGF-b1, TNF-a, IFN-r, and IL-6, respectively, to confirm the expression level of each inflammatory index, respectively.

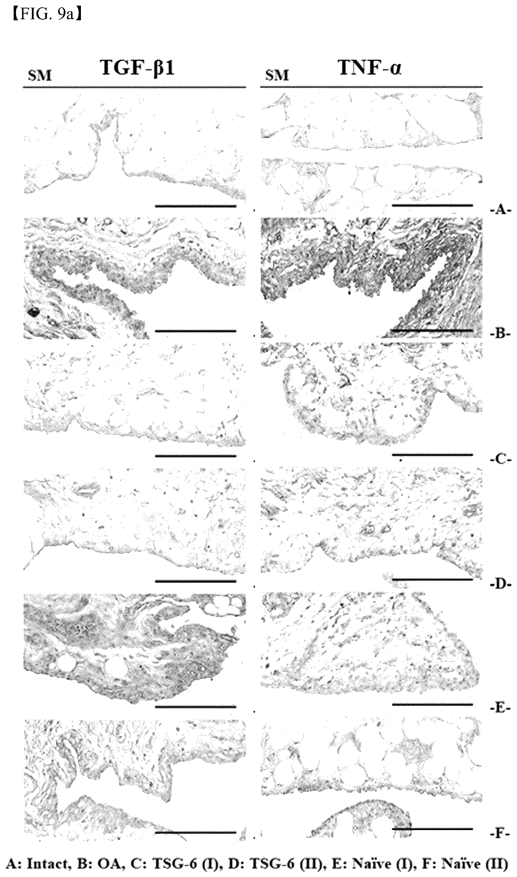

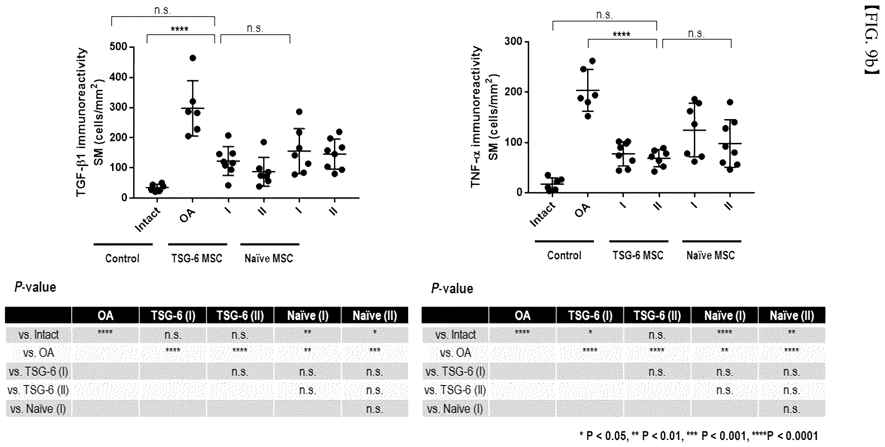

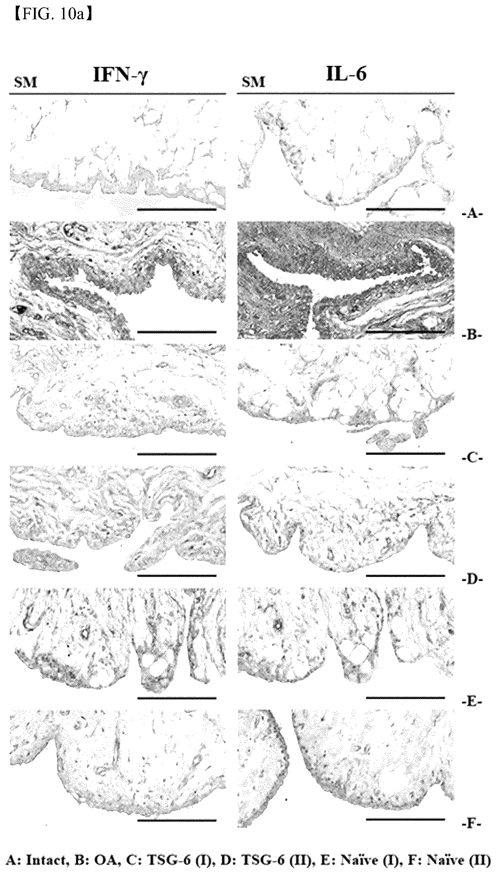

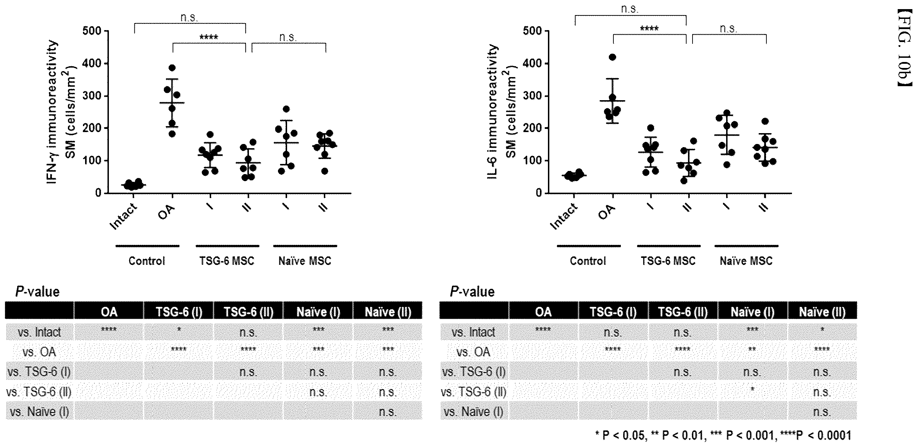

The IHC staining result for TGF-b1 and TNF-a by each group was shown in FIG. 9a, and the result of measuring the immunoreactivity of TGF-b1 and TNF-a by each group and the statistical significance were shown in FIG. 9b, and a graph of the result of measuring the immunoreactivity of IFN-r and IL-6 by each group and the statistical significance were shown in FIG. 10b. In Table 6 below, the result of measuring the expression level of inflammatory indexes by each group was shown.

**[Table 6]**

| Group | TGF-b1 | TNF-a | IFN-r | IL-6 |
|---|---|---|---|---|
| Intact | 34.33 | 17.17 | 25.33 | 54.67 |
| OA | 297.67 | 203.67 | 279.00 | 285.00 |
| TSG-6 I | 122.75 | 77.50 | 117.75 | 127.00 |
| TSG-6 II | 87.14 | 68.71 | 94.00 | 93.43 |
| Naive I | 155.43 | 124.86 | 156.00 | 180.29 |
| Naive II | 146.00 | 97.75 | 146.00 | 141.50 |

As the result of IHC staining for synovial cells, in the OA group, each inflammatory index was greatly increased, compared to Intact group, but in TSG-6 MSC administration groups (TSG-6 I, and TSG-6 II), all the inflammatory indexes were reduced. In particular, in TSG-6 II group, the immunoreactivity for TGF-b 1, IFN-r, and IL-6 was reduced to the extent similar to the normal group, and thus it was confirmed that the anti-inflammatory activity of TSG-6 MSC was excellent.

### Example 5. Comparison of expression vector of TSG-6 gene transduced MSC and TNF-a treated MSC

### 5-1. Preparation of TNF-a treated MSC

Cord blood-derived MSCs were activated with TNF-a. Briefly, the cord blood-derived MSCs were plated in an amount of 1×10⁵ cells in a 6-well plate including 2 ml Advanced MEM (Thermofisher, MA, US) medium comprising 10%(v/v) FBS per each well and cultured for 24 hours. Then, the medium was replaced with Advanced MEM medium including 1%(v/v) FBS and 10 ng/ml TNF-α (Peptrotech, NY, USA) and cultured for 24 hours. The cells were treated with 0.25%(w/v) trypsin together with 1 mM EDTA (Gibco) at 37°C for 2 minutes for trypsinization. The collected cells were used for the following example.

### 5-2. Comparison of TSG-6 mRNA level (Reverse transcription quantitative real-time PCR: RT-qPCR)

The TSG-6 expression level (mRNA level) of a naive cord blood-derived MSC (or less, `MSC'; control group), the TSG-6 expression vector transduced cord blood-derived MSC prepared in Example 1.2 (or less, `TSG-6 transduced MSC'; test group) and the cord blood-derived MSC activated with TNF-α prepared in Example 5-1 (or less, 'TNF-α treated MSC'; comparative group) for 24 hours was measured by RT-qPCR. According to the manufacturer's instructions using RNeasy Mini Kit (QIAGEN), the total RNA was extracted from the cells, and after synthesizing cDNA using SuperScript^{™} III First-Strand Synthesis System (Thermofisher), RT-qPCR was performed on QuantStudio^{™} 6 Flex Real-Time PCR System (Applied Biosystem) using Fast SYBR^{™} Green Master Mix (Thermofisher). As an endogenous control gene, GAPDH was used. The used primers were arranged in Table 7 below.

**[Table 7]**

| Gene | Forward sequence (5' to 3') | SEQ ID NO: | Reverse sequence (5' to 3') | SEQ ID NO: |
|---|---|---|---|---|
| TSG-6 | TGGATGGCTAAGGGCAGAG | 5 | GCGTGTGGGTTGTAGCA | 6 |
| GAPDH | GTCTCCTCTGACTTCAACAGCG | 7 | ACCACCCTGTTGCTGTAGCCAA | 8 |

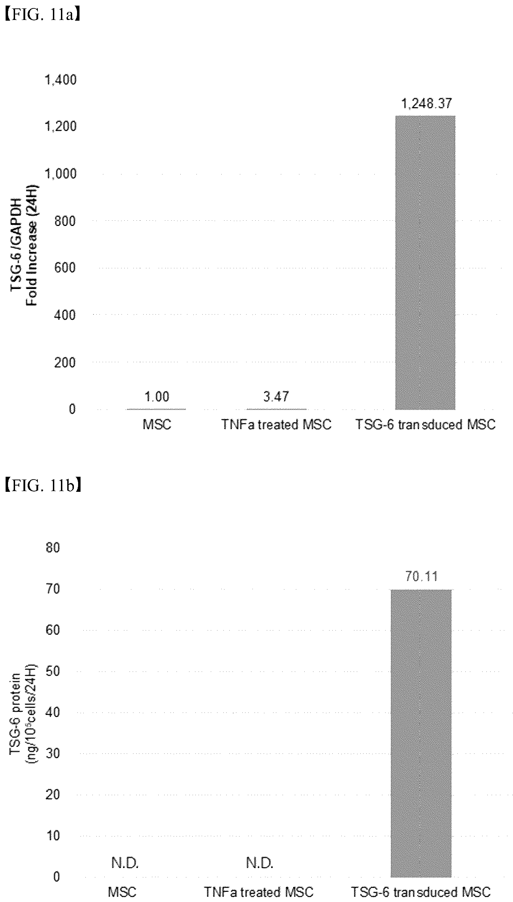

The obtained result was calculated as relative Ct values (TSG-6/GAPDH) and shown in FIG. 11a. The relative expression level was calculated using 2^{-ΔΔCt} method. As shown in FIG. 11a, the relative TSG-6 expression level (mRNA level) of TNF-α treated MSC (comparative group) did not show a big difference from the control group MSC (3.47 times compared to the control group), whereas the relative TSG-6 expression level (mRNA level) of TSG-6 transduced MSC (test group) was significantly higher than the comparative group as well as the control group (about 1248 times compared to the control group, about 360 times compared to the comparative group).

### 5-3. Comparison of TSG-6 protein level (ELISA)

The TSG-6 protein level expressed (secreted in a medium) in a naive cord blood-derived MSC (or less, `MSC'; control group), the TSG-6 expression vector transduced cord blood-derived MSC prepared in Example 1.2 (or less, `TSG-6 transduced MSC'; test group) and the cord blood-derived MSC activated with TNF-α prepared in Example 5-1 (or less, 'TNF-α treated MSC'; comparative group) for 24 hours was measured using TSG-6 ELISA kit (Raybiotech, GA, US) according to the manufacturer's instructions.

The obtained result (ng/10⁵cells/24 hours) was shown in FIG. 11b. As shown in FIG. 11b, the control group MSC and the comparative group TNF-α treated MSC had too low level of the TSG-6 protein secretion level to be measured, while the TSG-6 transduced MSC secreted TSG-6 protein at a significantly high level (about 70ng/10⁵cells/24 hours) compared to the control group MSC and the comparative group TNF-α treated MSC.

### 5-4. Comparison of collagen II mRNA level (RT-qPCR)

A naive cord blood-derived MSC (or less, `MSC'; control group), the TSG-6 expression vector transduced cord blood-derived MSC (or less, `TSG-6 transduced MSC'; test group; See Example 1.2) and the cord blood-derived MSC activated with TNF-α (or less, 'TNF-α treated MSC'; comparative group; See Example 5-1) were prepared, respectively. Each cord blood-derived MSC prepared as such was plated in an amount of 1×10⁵ cells in a 6-well transwell (Corning, MA, US) including 2 ml Advanced MEM medium comprising 10%(v/v) FBS per each well and cultured for 24 hours. Then, the medium was replaced with Advanced MEM medium including 1%(v/v) FBS and 10 ng/ml TNF-a and cultured for 24 hours to induce the activated MSC.

Chondrocytes (derived from costal cartilage; See Example 3) were plated in an amount of 1×10⁵ cells in a 6-well plate including 2 ml Advanced MEM medium comprising 10%(v/v) FBS per each well and cultured for 24 hours. Then, the medium was replaced with Advanced MEM medium including 1%(v/v) FBS and 75 ng/ml IL-1b (Peptrotech, NY, US) and cultured for 24 hours to induce inflammation. They were co-cultured with the MSC activated in the 6-well transwell for 24 hours. The chondrocytes were treated with 0.25%(w/v) trypsin together with 1 mM EDTA (Gibco) at 37°C for 2 minutes for trypsinization. The collected cells were used for the following comparison of the collagen II mRNA level (RT-qPCR).

The collagen II expression level (mRNA level) in chondrocytes co-cultured with the naive cord blood-derived MSC (MSC; control group), the TSG-6 expression vector transduced cord blood-derived MSC (TSG-6 transduced MSC; test group) or the cord blood-derived MSC activated with TNF-α (TNF-α treated MSC; comparative group) for 24 hours was measured by RT-qPCR. According to the manufacturer's instructions using RNeasy Mini Kit (QIAGEN), the total RNA was extracted from the cells, and after synthesizing cDNA using SuperScript^{™} III First-Strand Synthesis System (Thermofisher), RT-qPCR was performed on QuantStudio^{™} 6 Flex Real-Time PCR System (Applied Biosystem) using Fast SYBR^{™} Green Master Mix (Thermofisher). As an endogenous control gene, GAPDH was used. The used primers were arranged in the Table 8.

**[Table 8]**

| Gene | Forward sequence (5' to 3') | SEQ ID NO: | Reverse sequence (5' to 3') | SEQ ID NO: |
|---|---|---|---|---|
| collagen II | CTCAAGTCGCTGAACAACCA | 9 | GTCTCCGCTCTTCCACTCTG | 10 |
| GAPDH | GTCTCCTCTGACTTCAACAGCG | 7 | ACCACCCTGTTGCTGTAGCCAA | 8 |

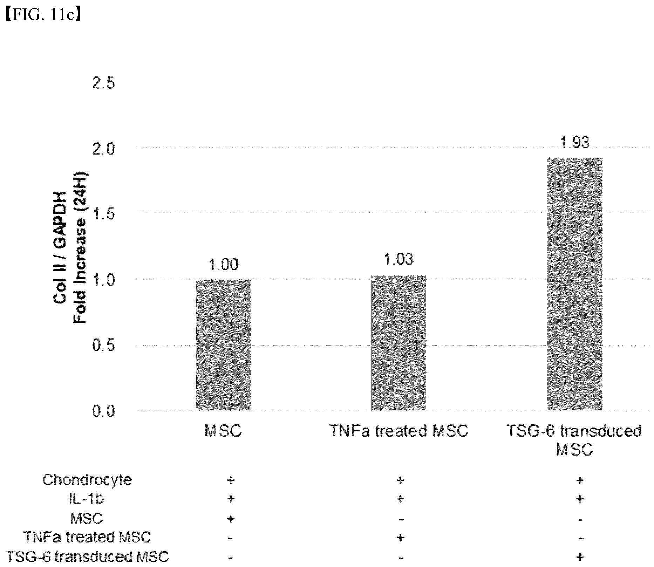

The obtained result was calculated as relative Ct values (Collagen II/GAPDH) and shown in FIG. 11c. The relative expression level was calculated using 2^{-ΔΔCt} method. As shown in FIG. 11c, the relative collagen II expression level (mRNA level) of TNF-α treated MSC (comparative group) did not show a big difference from the control group MSC (1.03 times compared to the control group), whereas the relative collagen II expression level (mRNA level) of TSG-6 transduced MSC (test group) was significantly higher than the comparative group as well as the control group (about 1.93 times compared to the control group, about 1.87 times compared to the comparative group). This result confirmed that the TSG-6 expression vector transduced MSC had a significantly excellent collagen II expression effect in chondrocytes, compared to the naive MSC as well as the TNF-α activated (inducible) MSC, and this result demonstrates the excellent cartilage regeneration effect and/or osteoarthritis treatment effect of the TSG-6 expression vector transduced MSC.

## Claims

1. A composition for cartilage regeneration, comprising a mesenchymal stem cell expressing a TSG-6 protein as an active ingredient.

2. The composition for cartilage regeneration according to claim 1, wherein the mesenchymal stem cell expressing TSG-6 protein is a recombinant mesenchymal stem cell comprising one or more selected from the group consisting of a gene encoding TSG-6 protein and a recombinant vector comprising the gene encoding TSG-6 protein.

3. The composition for cartilage regeneration according to claim 1, wherein the composition has
an expression level of the TSG-6 protein is 10 to 200 ng/100,000 cells/24 hours,
an expression level of the TSG-6 protein is 10 to 50,000 ng/mL/24 hours, or
both.

4. The composition for cartilage regeneration according to claim 1, wherein an expression level of collagen II protein in at least one selected from the group consisting of a chondrocyte and cartilage tissue administered with the composition, is increased compared to a non-administered control group.

5. The composition for cartilage regeneration according to claim 1, wherein
an expression level of an inflammatory index in at least one selected from the group consisting of a chondrocyte, cartilage tissue, a synoviocyte and synovial tissue administered with the composition, is reduced compared to a non-administered control group, and
the inflammatory index is at least one selected from the group consisting of TGF-b 1, TNF-a, IFN-r, and IL-6.

6. A pharmaceutical composition for prevention or treatment of osteoarthritis, comprising a mesenchymal stem cell expressing a TSG-6 protein as an active ingredient.

7. The pharmaceutical composition for prevention or treatment of osteoarthritis according to claim 6, wherein the mesenchymal stem cell expressing TSG-6 protein is a recombinant mesenchymal stem cell comprising one or more selected from the group consisting of a gene encoding TSG-6 protein and a recombinant vector comprising the gene encoding TSG-6 protein.

8. The pharmaceutical composition for prevention or treatment of osteoarthritis according to claim 7, wherein the composition has
an expression level of the TSG-6 protein is 10 to 200 ng/100,000 cells/24 hours,
an expression level of the TSG-6 protein is 10 to 50,000 ng/mL/24 hours, or
both.

9. The pharmaceutical composition for prevention or treatment of osteoarthritis according to claim 7, wherein the composition has an effect of:
increasing an expression level of collagen II protein in at least one selected from the group consisting of a chondrocyte and cartilage tissue compared to a non-administered control group,
decreasing an expression level of at least one inflammatory index selected from the group consisting of TGF-b1, TNF-a, IFN-r, and IL-6 in one or more selected from the group consisting of a chondrocyte, cartilage tissue, a synoviocyte and synovial tissue, compared to a non-administered control group, or
both.

10. The pharmaceutical composition for prevention or treatment of osteoarthritis according to any one of claim 6 to claim 9, which is for intra-articular administration.

11. The pharmaceutical composition for prevention or treatment of osteoarthritis according to any one of claim 6 to claim 9, which is an injectable agent.

12. A composition for expressing collagen II protein, comprising a mesenchymal stem cell expressing a TSG-6 protein as an active ingredient.

13. The composition for expressing collagen II protein according to claim 12, wherein the mesenchymal stem cell expressing TSG-6 protein is a recombinant mesenchymal stem cell comprising one or more selected from the group consisting of a gene encoding TSG-6 protein and a recombinant vector comprising the gene encoding TSG-6 protein.

14. The composition for expressing collagen II protein according to claim 12, wherein the composition has
an expression level of the TSG-6 protein is 10 to 200 ng/100,000 cells/24 hours,
an expression level of the TSG-6 protein is 10 to 50,000 ng/mL/24 hours, or
both.
